# EUROPEAN PATENT APPLICATION

(11) **EP 4 265 272 A1**
(43) Date of publication of application: **25.10.2023**
(21) Application number: 21854867.5
(22) Date of filing: 14.12.2021
(51) Int. Cl.: A61K 39/12

(54) **USE OF VACCINE COMPOSITIONS BASED ON SARS-COV-2 RECEPTOR BINDING DOMAIN IN DELIVERING PROTECTIVE IMMUNITY**

(30) Priority: 16.12.2020 CU 20200101
(71) Applicant: INSTITUTO FINLAY DE VACUNAS, La Habana, C.P. 11600 (CU); Centro de Inmunologia Molecular, La Habana, Habana 11300 (CU)
(72) Inventor: VEREZ BENCOMO, Vicente Guillermo, 11300 La Habana (CU); VALDÉS BALBÍN, Yury, 11300 La Habana (CU); GARCÍA RIVERA, Dagmar, 17100 La Habana (CU); OCHOA AZZE, Rolando, 10400 La Habana (CU); CLIMENT RUIZ, Yanet, 10400 La Habana (CU); GONZÁLEZ RODRÍGUEZ, Humberto, 11500 La Habana (CU); OROSA VAZQUEZ, Ivette, La Habana (CU); DÍAZ HERNÁNDEZ, Mariannis, 20100 Pinar del Río (CU); SÁNCHEZ RAMÍREZ, Belinda, 11300 La Habana (CU); OJITO MAGAZ, Eduardo, 11300 La Habana (CU); LEÓN MONZÓN, Kalet, 17100 La Habana (CU); MACIAS ABRAHAM, Consuelo Milagro, 10400 La Habana (CU); CHANG MONTEAGUDO, Arturo, 11300 La Habana (CU); PORTO GONZÁLEZ, Delia Esther, 10900 La Habana (CU); DUBED ECHEVERRÍA, Marta, 10900 La Habana (CU); RODRÍGUEZ ACOSTA, Mireida, 11300 La Habana (CU)
(74) Representative: V.O.
(86) International application number: PCT/CU2021/050014
(87) International publication number: WO 2022/127946

(57) **Abstract**

This invention relates to the field of Biotechnology and Medicine. It describes the use of vaccine compositions based on the receptor binding domain of SARS-CoV-2 virus in the treatment of patients recovered from COVID-19 and in subjects vaccinated with vaccine platforms other than subunit vaccines, who fail to develop effective protective immunity or where immunity has decreased over time and a booster with the same vaccine used in primary vaccination is not recommended. Particularly, this use is described for vaccine compositions comprising a covalent conjugate of the receptor binding domain (RBD) and a carrier protein such as tetanus toxoid, diphtheria toxoid and diphtheria toxoid mutant CRM197, vaccine compositions having the RBD as antigen, with or without the immunopotentiator outer membrane vesicles of serogroup B *Neisseria meningitidis.*

## Description

### TECHNICAL FIELD

This invention relates to the field of Biotechnology and Medicine, particularly to the use of vaccine compositions based on the SARS-COV-2 Receptor Binding Domain in the treatment of patients recovered from COVID-19 and vaccinated subjects who failed to develop protective immunity, in which a significant natural protective antibody response has decreased or was not induced.

### TECHNOLOGICAL BACKGROUND

COVID-19 is a very recent disease, discovered in Wuhan, China in December 2019, when serious cases of pneumonia of unknown etiology began to be reported. The disease caused by the SARS-CoV-2 virus is characterized by its fast spreading and the appearance of symptoms such as fever, cough, rhinorrhea, a sore throat and dyspnea in the case of symptomatic patients, who account for less than 50 %. The rest of the people infected with the disease are asymptomatic, which is a key factor in the spreading of the virus and represents an epidemiological challenge in terms of its control (WHO Coronavirus disease (COVID-2019) situation reports. https://www.who.int/emergencies /diseases/novel-coronavirus-2019/situation-reports. Consulted on 13 August 2020).

Other coronaviruses similar to SARS-CoV-2, known as MERS and SARS, have already caused similar epidemics in previous decades. SARS shows greater homology with SARS-CoV-2, and one of the main similarities between them is that both viruses use the ACE2 protein as a receptor to penetrate human cells. Therefore, in both SARS and SARS-CoV-2, the interaction between the receptor binding domain (RBD) of the S1 viral protein and the ACE2 (*angiotensin-converting enzyme 2*) protein is a decisive factor for humans to get infected with the virus. (Walls A et al (2020) Cell https://doi.org/10.1016/j.cell.2020.02.058).

The RBD of the S protein of the SARS-CoV-2 is a fragment of approximately 195 amino acids (sequence 333-257), which contains the receptor binding motive (RBM) and is the region in which the virus interacts with the ACE2 receptor. The RBD contains 4 intramolecular disulfide bridges between cysteines Cys336-Cys361, Cys379-Cys432, Cys391-Cys525, and Cys480-Cys488, which helps create a very compact and stable structure (Lan et al (2020), Nature https://doi.org/10.1038/s41586-020-2180-5).

The RBD is a small molecule, whose molecular mass ranges from 25 to 27 kDa depending on the expression host and the carbohydrates incorporated, mainly linked to asparagines N331 and N343 (Chen WH et al,2017, Journal of Pharmaceutical Sciences 106: 1961-1970).

Strategies for SARS-Cov-2 vaccines include the inactivated virus, genetic constructions that contain viral genetic material incorporated in an adenovirus or as messenger RNA, and vaccines based on viral protein subunits or fragments expressed in genetically modified hosts.. In this case, the molecule preferred is the S protein, also known as Spike protein, or a fragment of its structure, i.e., the RBD. Their main advantage is their safety, , as this strategy is closer to that of many vaccines in use ; nevertheless, its main challenge is the achievement of an immune response that is sufficient to protect from viral infection.

As of December 2, 2020, 163 vaccine candidates against SARS-CoV-2 were in preclinical evaluation and 51 in clinical trials. Of this number, there are at least 13 vaccine candidates (5 in clinical trials and 8 in preclinical trials) with RBD as the specific antigen (DRAFT Landscape of COVID-19 Candidate Vaccines - December 2, 2020, available at:https://www.who.int/publications/m/item/draft-landscape-of-covid-19-candidate-vaccines).

Several months after the start of the pandemic, the impact on the immune system of patients of COVID-19 is being better understood. The disease, especially in its severe and critical stages, has a strong hyper-inflammatory component that triggers a cytokine storm (de la Rica, R; Borges M; Gonzalez-Freire, M (2020) Front. Immunol. https://doi.org/10.3389/fimmu.2020.558898; Riva et al. (2020) Critical Care. 24:549), which can contribute to lethality if not properly controlled. (Tisoncik, J et al. (2012) Microbiology and Molecular Biology. 76(1):16-32). In fact, several drugs are being developed for this purpose, some of which are being introduced in COVID-19 treatment protocols in Cuba (Martinez et al. (2020) Anales de la Academia de Ciencias de Cuba. 10(2) http://www.revistaccuba.sld.cu, consulted on December 2, 2020) and in the world (Xu, X et al. (2020) Military Medical Research. 7:22).

Moreover, several studies have shown anti-RBD antibodies neutralizing SARS-CoV-2, in patients recovered from the disease (convalescents). Antibody levels vary among individuals, with a certain correlation between their titers and the severity of the disease (lower in asymptomatic patients than in patients with moderate, severe or critical disease). (Seow, J et al. (2020) Nature Microbiology doi: 10.1038/s41564-020-00813-8; Bošnjak, B et al. (2020) Cell Mol Immunol. doi: 10.1038/s41423-020-00573-9). It has also been documented that the anti-RBD antibody titer in convalescents correlates strongly with the capacity to neutralize SARS-CoV-2 viral infection of *in vitro* cells (Tan, C et al. (2020) Nature Biotechnology https://doi.org/10.1038/s41587-020-0631-z). Nevertheless, recent studies have shown that the anti-RBD antibody titer and its SARS-CoV-2 neutralizing capacity is time-dependent and progressively reduced in individuals recovered from infection. (Lee, W et al. (2020) The Journal of Infectious Diseases, DOI: 10.1093/infdis/jiaa673). In addition, there is increasing evidence of reinfection of patients, even with cases where the second infection has been more severe than the first. (Qu, YM and Cong HY (2020) Travel Med Infect Dis. 34:101619; Lan L et al. (2020) JAM. 323:1502-3; Tillett, R et al. (2020) Lancet Infect Dis. https://doi.org/10.1016/S1473-3099(20)30764-7). These data have drawn the attention of the international scientific community to the need for strategies for their effective protection. (Overbaugh, J (2020) Nature, 26 1678-1685).

Given that the immune system of SARS-CoV-2 convalescents had encountered viral antigens in a very peculiar *(sui-generis)* immunological context, it is not evident that the vaccines or vaccine strategies developed for protecting naive individuals who have not been in contact with the virus are appropriate or effective in achieving or enhancing protection against reinfection, without generating unintended adverse effects such as a reactivation of the cytokine storm or the stimulation of antibody production, causing the phenomenon known as ADE (*antibody dependent enhancement*) (Arvin, AM et al. (2020) Nature 584(7821):353-363). In addition, the length of the induced protection provided by the various vaccine technologies in use for COVID-19 is not known, and if a booster dose is required, some of these vaccines could not be used as a booster. This invention describes for the first time the use of vaccine compositions based on the SARS-CoV-2 receptor binding domain in the treatment of COVID-19-recovered patients in which a protective antibody response has decreased or was not induced. Additionally, these vaccine compositions can be used in individuals previously vaccinated with an adenovirus or RNA vaccines. The vaccine compositions described are also being tested in uninfected individuals, but now for the first time they demonstrate their ability to efficiently re-stimulate in convalescent patients a strong immune response with a neutralizing capacity. The fact that both vaccine strategies are based on a small recombinant protein of the SARS-CoV-2 virus, the RBD, may give a special advantage in this field of application. In particular, they trigger a highly targeted response against RBM, responsible of the interaction of the virus with the ACE2 receptor in host cells. This highly targeted response, with a satisfactory safety profile, is an advantage when compared to other vaccine strategies such as attenuated viral vaccines, S protein-based vaccines or viral vector vaccines.

### BRIEF DESCRIPTION OF THE INVENTION

In one embodiment, this invention relates to the use of vaccine compositions comprising the receptor binding domain (RBD) of the SARS-CoV-2 virus in the treatment of recovered COVID-19 patients. Particularly, these recovered patients have a humoral immunity characterized by-at least- one of the following conditions: the response titer against RBD is less than 1:1,000, the inhibitory capacity of the RBD-ACE2 interaction is less than 50% in a 1:100 dilution, or SARS-CoV-2 neutralizing antibody titer is less than 1:160.

In a particular embodiment, this invention relates to the above mentioned use of vaccine compositions characterized by comprising a covalent conjugate of the RBD and a carrier protein selected from the group consisting of: tetanus toxoid, diphtheria toxoid and diphtheria toxoid mutant CRM197. Other vaccine compositions in the claims are those comprising the RBD as antigen, either in its monomeric or dimeric form, adsorbed to Al(OH)₃. Other claimed vaccine compositions comprise the use of immunopotentiators such as the *Neisseria meningitidis* outer membrane vesicle.

In a particular embodiment, the above vaccine compositions are administered intramuscularly or subcutaneously to recovered COVID-19 patients in an immunization schedule comprising one to three doses of 1 to 100 µg of RBD at intervals of 21 to 28 days. This invention is intended for use according to the above immunization schedule to obtain hyperimmune plasma with high SARS-CoV-2 neutralizing capacity.

In another embodiment, this invention covers the use of the vaccine compositions referred to herein in the immunization of subjects vaccinated with vaccine platforms other than subunit vaccines and who have not developed an effective protective immunity or where this protective immunity has decreased over time and a booster dose is not recommended with the same vaccine used in primary immunization, such as the adenovirus, inactivated virus, attenuated virus and mRNA vaccines. According to this invention, effective protective immunity is achieved where at least one of the following conditions applies: response titer against RBD is greater than 1:1,000, inhibitory capacity of the RBD-ACE2 protein interaction is greater than 50% at a 1:100 dilution or SARS-CoV-2 neutralizing antibody titer is greater than 1:160.

### DETAILED DESCRIPTION OF THE INVENTION

This invention describes the use of vaccine compositions based on the SARS-CoV-2 virus receptor binding domain in COVID-19 recovered (convalescent) patients, without significant levels of natural anti-RBD antibodies with neutralizing capacity. Particularly, these vaccine compositions may contain dimeric and/or monomeric forms of RBD adsorbed to Al(OH)₃, with or without an immunopotentiator, or be more complex as detailed in those included, but not limited to, patent applications CU-2020-0057 and CU-2020-0069.

For use in COVID-19 convalescents, it must be verified that at least one of the following conditions applies: anti-RBD antibody titer is less than 1:1,000 as measured by ELISA, or the SAR,S-CoV-2 infectivity neutralizing antibody titer is less than 1:160 as measured in assays with live-virus or pseudovirus, or the capacity for inhibiting RBD-ACE2 interaction protein is less than 50% at a 1:100 dilution of serum as measured in a competitive ELISA .

Vaccine preparations induce activation of the memory immune response to the virus, ensuring high RBD antibody titers, mostly with neutralizing capacity for several months (at least 6 months). If the patient's titers decline again, an additional booster dose may be administered to regain protection. Given their broad safety, vaccine preparations can be administered for several consecutive years, from 2 to 5 years. In addition, the vaccine compositions referred to in this invention can be used in the immunization of subjects vaccinated with vaccine platforms other than subunit vaccines, who have not developed effective protective immunity, or once immunity has decreased and a booster dose is required. It has been documented that adenoviral vector-based vaccine platforms can only immunize subjects once or twice, because after the first injection, antibodies against the adenoviral vector itself are produced that may render the second dose ineffective. (Casimiro, DR et al. (2003) J. Virol., 77: 7663-7668). Vaccine preparations are administered to convalescent patients intramuscularly or subcutaneously in an immunization schedule comprising a range of one to three doses of 1 to 100 µg of RBD, preferably between 30 and 60 µg at intervals of 21 to 28 days.

This invention further comprises a method for obtaining hyperimmune plasma with SARS-CoV-2 virus neutralizing capacity from antibodies produced by COVID-19 convalescents after vaccination. Hyperimmune plasma is useful for the treatment of patients with moderate, severe or critical COVID-19, as highlighted by several authors (Bloch EM et al. (2020) J Clin Invest. 130:2757-65, Casadevall A. (2020) JAMA 324:455-7). Donors are required to be symptom-free, tested negative for SARS-CoV-2 real-time PCR and have neutralizing antibody titers of at least 1:160, according to the recommendations of the FDA (US Department of Health and Human Services Food and Drug Administration. Investigational COVID-19 convalescent plasma: guidance for industry. Rockville, MD: FDA, 2020). The method of this invention allows obtaining hyperimmune plasma from convalescents who have not spontaneously generated a good immune response. To this end, convalescents are immunized according to the immunization schedule described herein until the desirable level of neutralizing antibodies (titers >1:160) are reached for obtaining the hyperimmune plasma useful for the passive transfer of antibodies against SARS-CoV-2. The plasma to be used in this method will be processed according to the blood processing standards for such purposes.

### BRIEF DESCRIPTION OF FIGURES

**Figure 1****.** Correlation between total IgG antibody titers detected against the RBD and inhibition values in percentage for sera at 1:100 dilution.
**Figure 2****. A:** Inhibition values in percentage of the RBD-ACE2 interaction of sera from subjects six months after receiving immunization scheme with mRNA vaccine and after applying a booster dose with RBD vaccine. B: Concentration of anti-RBD antibodies detected in subjects immunized with inactivated virus vaccine who received booster doses with RBD vaccine.
**Figure 3****.** Concentration of anti-RBD antibodies detected in subjects immunized with RBD vaccine who received booster doses with RBD conjugate vaccine.

### EXAMPLES

### Example 1. Variability of anti-RBD titers and inhibitory capacity of the RBD-ACE2 interaction in Cuban patients recovered from COVID-19.

Serum from 39 COVID-19 convalescent individuals is analyzed in an indirect ELISA to determine the anti-RBD antibody titer. NUNC MaxiSorp 96-well microtiter plates were coated with 50 µL of RBD, 5 µg/mL in phosphate-buffered saline solution (PBS, pH 7.0), incubated for 1 hour at 37°C. Uncoated sites were blocked with 150 µL of a blocking solution (PBS, Tween 20 0.05% [v:v], 4% skim milk) for 30 minutes at 37°C. Sera dissolved in blocking solution (100 µL/well) were then added in serial dilutions (1:2), generally starting from 1:100.. The plates were incubated overnight at 4°C and washed three times with PBS, Tween 20 (0.05%) [v:v]. A dilution of peroxidase-conjugated human anti-immunoglobulin G (100 µL) was added in blocking solution (1:5000) and incubated for 1 hour at room temperature. After a final washing step, peroxidase enzyme substrate solution (100 µL/well) was added. It was incubated in the dark for 20 minutes and the reaction was stopped with H₂SO₄ 2N (50 µL/well). Absorbance was read at 490 nm using an ELISA reader. In order to determine the IgG titer, a linear regression was performed in the assessed range of dilutions and interpolated. The threshold value was twice the mean absorbance of a negative serum obtained prior to COVID-19, diluted 1:100.

Sera from these same 39 convalescents were evaluated in an ELISA to determine their capacity to inhibit the RBD-ACE2 interaction. Plates coated with human ACE2-Fc (5 µg/mL) were blocked and the mouse RBD-Fc fusion was added with serum from convalescents, at dilutions from 1:100 to 1:10,000, previously incubated for 1 hour at 37°C. An anti-mouse IgG-alkaline phosphatase conjugate, diluted in milk 0.2% and SSTF-T was used for recognition detection. After a final washing step, 50 µL/well of pNPP in diethanolamine buffer (1mg/mL) was applied. It was incubated in the dark for 30 minutes and the reaction was stopped with 3M NaOH (50 µL/well). Absorbance was read at 405 nm. The percentage of inhibition was calculated using the following formula: (1-Abs405nm mouse RBD Fc + convalescent serum/Abs405nm mouse RBD Fc) *100.

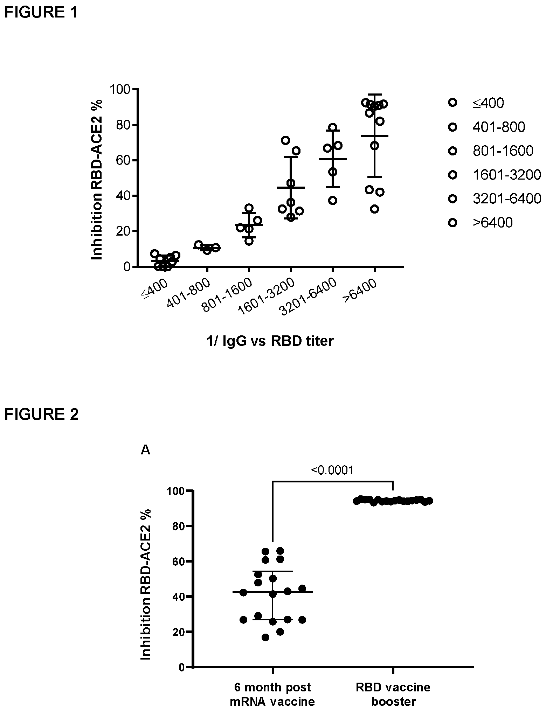

Figure 1 shows the inhibitory capacity of sera from convalescent individuals (n=39), at a dilution of 1:100 as a function of the antibody titer against RBD. A positive Spearman's correlation (r=0.9178) is observed between total antibody titers against RBD and their inhibitory capacity, r=0.9178. Of all individuals tested, 41% had inhibition values below 30% and anti-RBD antibody titers below 1:800.

The high variability observed in Cuban convalescents, both in anti-RBD antibody titers and in their capacity to inhibit the RBD-ACE2 interaction, is consistent with data reported in the literature (Tan, C et al. (2020) Nature Biotechnology https://doi.org/10.1038/s41587-020-0631-z). Literature also confirms that in COVID-19 convalescents there is a positive correlation between anti-RBD IgG titers and the capacity of serum/plasma to neutralize live virus infection. Only high anti-RBD antibody titers (greater than 1:1350) foresee a higher probability (80%) of neutralizing capacity at 1:160 dilutions. (Salazar, E et al. (2020) bioRxiv. https://doi.org/10.1101/2020.06.08.138990).

### Example 2. COVID-19 convalescent patients who do not develop effective humoral immunity achieve protection after immunization.

Blood samples were taken from two patients recovering from COVID-19 at 2 and 4 months after a negative RT-PCR test for the detection of SARS-CoV-2. Serum was tested for anti-RBD antibody titer and inhibitory capacity of the RBD-ACE2 interaction in an ELISA described for such purposes in Example 1 and for SARS-CoV-2 neutralizing antibody titer by a colorimetric neutral red assay described in patent application CU-2020-0069. The above techniques are detailed in patent applications CU-2020-0057 and CU-2020-0069.

After verifying that in both cases the anti-RBD antibody titer and the neutralizing antibody titer were below the parameters established for a protective response, both patients were immunized with 50 µg of dimeric RBD absorbed on Al(OH)s. On day 14 after immunization, blood was collected. From the serum obtained, the anti-RBD antibody titer and the inhibitory capacity of the RBD-ACE2 interaction and SARS-CoV-2 neutralizing antibody titer were again determined.

Table 1 shows outcomes in the two patients for the determinations performed at day zero (before immunization) and day 14 after immunization. As can be noted, after a single immunization both patients had high anti-RBD antibody titers, inhibition values greater than 80% for RBD-ACE2 interaction and neutralizing antibody titers much greater than 1:160. It is important to note that the increase in neutralizing titers is higher than the increase in anti-RBD titers, which proves that this immunization preferentially enriches the serum/plasma in antibodies with a neutralizing capacity.

**Table 1. Determinations in COVID-19 convalescent patients before and after immunization.**

| | **Anti-RBD titer** | | | **Inhibitory capacity of RBD-ACE2 interaction** | | **Neutralizing antibody titer** | | |
|---|---|---|---|---|---|---|---|---|
| Time (day) | T0 | T14 | Growth | T0 | T14 | T0 | T14 | Growth |
| Subject 1 | 1/800 | 1/12800 | x16 | 48% | 95% | 1/54 | 1/2560 | x47 |
| Subject 2 | 1/400 | 1/3200 | x8 | 35% | 96% | 1/40 | 1/3162 | x79 |

It is also important to emphasize that a satisfactory safety profile was noted in both individuals. No significant adverse events occurred.

### Example 3. Increase in concentration of anti-RBD antibodies and inhibitory capacity of the RBD-ACE2 interaction in subjects previously immunized with inactivated virus or mRNA vaccine who receive booster doses with a vaccine composition comprising RBD.

Subjects who received the complete scheme of two doses of the inactivated virus or mRNA vaccine had their blood drawn six months later to evaluate the duration of the response generated by the first vaccination. These subjects received a dose of vaccine compositions based on the RBD of the SARS-COV Virus and the concentration of anti-RBD antibodies and the inhibitory capacity of the ACE2 RBD-protein interaction were evaluated between 14 and 28 days after the booster dose.

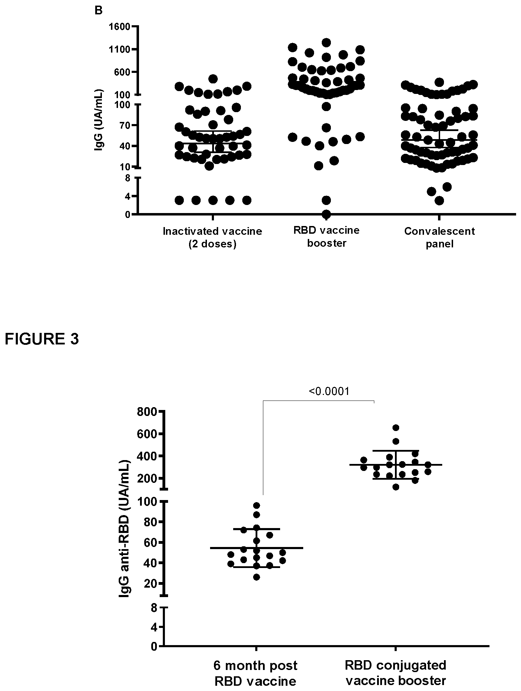

Figure 2 shows the inhibitory capacity of sera from individuals vaccinated with mRNA vaccine and who received a booster dose of a vaccine composition containing the RBD with outer membrane vesicle of Neisseria meningitidis adsorbed to AIOH₃ (Fig. 2A). As can be seen, after six months of receiving the complete scheme, the inhibition values are below 65%, and after immunization with the booster dose these values increase above 90% for all subjects evaluated. A similar increase is observed when applying a vaccine composition containing the RBD adsorbed to AIOH₃ in subjects previously immunized with two doses of inactivated virus vaccine. The sera were evaluated 21 days after applying the complete scheme of the inactivated virus vaccine and anti-RBD antibody concentration values were observed with a mean of 40 AU/mL that increased to more than 200 AU/mL after applying the vaccine. booster dose.

In both cases, the ability to reinforce pre-existing immunity was demonstrated when applying a dose in previously vaccinated subjects.

### Example 4. Increase in the concentration of anti-RBD antibodies in subjects previously immunized with RBD vaccine, who receive booster doses with a vaccine composition comprising RBD conjugated to tetanus toxoid.

Subjects immunized with two doses of an RBD-based vaccine candidate received a booster dose of a vaccine composition comprising a covalent conjugate between RBD and tetanus toxoid as carrier protein at 6 months. As can be seen in Figure 3, all the subjects showed an increase in the antibody concentration values after applying a booster dose with the vaccine composition containing the conjugated RBD.

## Claims

1. Use of a vaccine composition comprising the receptor binding domain (RBD) of the SARS-CoV-2 virus in the treatment of recovered COVID-19 patients.

2. Use according to claim 1 wherein recovered patients have a humoral immunity **characterized by** at least one of the following conditions:
- response titer against RBD is less than 1: 1000,
- inhibitory capacity of RBD-ACE2 protein interaction is less than 50% at a 1:100 dilution or
- SARS-CoV-2 neutralizing antibody titer is less than 1:160.

3. Use according to claim 1 wherein the vaccine composition is **characterized by** comprising a covalent conjugate of the RBD and a carrier protein which is selected from the group consisting of:
- Tetanus toxoid,
- Diphtheria toxoid and
- CRM197.

4. Use according to claim 1 wherein the vaccine composition is **characterized in that** the RBD is adsorbed to Al(OH)s as antigen.

5. Use according to claim 4 **characterized in that** the vaccine composition additionally comprising an immunopotentiator.

6. Use according to claim 5 **characterized by** having the *Neisseria meningitidis* outer membrane vesicle as immunopotentiator.

7. Use according to claim 4 wherein the RBD is in monomer form.

8. Use according to claim 4 wherein the RBD is in dimer form.

9. Use according to any one of claims 1-8 wherein the vaccine compositions are administered intramuscularly or subcutaneously to convalescent patients in an immunization schedule comprising a range of one to three doses between 1 and 100 µg of RBD, at intervals of 21 to 28 days.

10. Use according to claim 9 to obtain hyperimmune plasma with a high SARS-CoV-2 neutralizing capacity.

11. Use of a vaccine composition comprising the SARS-CoV-2 RBD for delivering effective protective immunity in subjects previously immunized with vaccines that are selected from the group consisting of:
- Adenovirus,
- Inactivated virus
- Attenuated virus
- mRNA -based vaccines

12. Use according to claim 11 wherein protective immunity is considered effective where at least one of the following conditions applies:
- response titer against the RBD is greater than 1:1000,
- the inhibitory capacity of RBD-ACE2 interaction is greater than 50% at a 1:100 dilution, or
- SARS-CoV-2 neutralizing antibody titer is greater than 1:160.

13. Use according to claim 11 wherein the vaccine composition is **characterized by** a covalent conjugate between the RBD and a carrier protein which is selected from the group consisting of:
- Tetanus toxoid,
- Diphtheria toxoid and
- Diphtheria toxoid mutant
- CRM197.

14. Use according to claim 11 wherein the vaccine composition is **characterized in that** the RBD is absorbed on Al(OH)s as antigen.

15. The vaccine composition according to claim 14 **characterized by** additionally comprising an immunopotentiator.

16. The vaccine composition according to claim 15 **characterized by** having the *Neisseria meningitidis* outer membrane vesicle as immunopotentiator.

17. Use according to claim 14 wherein the RBD is in monomer form.

18. Use according to claim 14 wherein the RBD is in dimer form.
